# EUROPEAN PATENT APPLICATION

(11) **EP 1 103 263 A2**
(43) Date of publication of application: **30.05.2001**
(21) Application number: 00310132.6
(22) Date of filing: 15.11.2000
(51) Int. Cl.: A61K 31/765

(54) **Poly-L-lactates as antitumour agents**

(30) Priority: 15.11.1999 JP 35960399
(71) Applicant: Nagasu, Youichiro, Fukuoka 827-0004 (JP)
(72) Inventor: Nagasu, Youichiro, Tagawa-gun, Fukuoka 827-0004 (JP); Aizawa, Shin, 176-001 Tokyo (JP); Nagasu, Masaya, Tokyo 125-0061 (JP); Imanishi, Yoshio, Ashigarashimo-gun, Kanagawa 259-0304 (JP)
(74) Representative: Silverman, Warren

(57) **Abstract**

The anti-malignant tumor agent of the present invention comprises a mixture of poly L-lactates that are cyclic and linear oligomers having degrees of condensation of 3 to 23, which is prepared by dehydration condensation of L-lactic acid in a nitrogen atmosphere under stepwise reduced pressure and at an increasing temperature, drying ethanol- and methanol-soluble components in the resulting reaction solution under reduced pressure, subjecting the product to chromatography on a reverse phase ODS column, and after elution with 25 to 50 % aqueous acetonitrile at pH 2.0, eluting said mixture of poly L-lactates with 100 % acetonitrile at pH 2.0.

## Description

The present invention relates to an anti-malignant tumor agent used in malignant neoplasms including cancers in mammals including humans.

As therapies against malignant neoplasms, surgical therapy, chemotherapy and radiotherapy have been conducted as three major therapies, but they still do not attain satisfactory effects.

In particular, chemotherapy has a generally strong action and is used frequently for a long time, and thus shows very strong side effects, and the secondary transformation caused by an anti-tumor agent is also problematic in recent years.

Further, effective chemicals have not been developed until now for some types of tumor. Accordingly, therapy by combined use of plural chemicals has been attempted to suppress side effects and to improve anti-tumor effects, but these problems are still not solved. In addition, developments of new immunotherapy and gene therapy are advanced, but these are still in the experimental stage.

As to the administration route of chemicals, many chemicals are given by intravascular administration, and the number of highly effective anti-tumor agents for oral administration is limited. That is, because intravascular administration is a limitation on use, chemotherapy is conducted usually under the control of the doctor in hospital thus causing mental problems or the problems of economical burden on the patient and difficult returning to work for long hospitalization, and there is demand for development of effective anti-tumor agents such as oral agents which are usable for outpatients.

A Japanese patent document, JP-A 5-310581, discloses a malignant tumor cell growth inhibitor for mammals including humans comprising a mixture of a L-lactate linear polycondensate with a degree of condensation of 5 to 23 and a L-lactate cyclic polycondensate with a degree of condensation of 9 to 19, which is obtained by:
(a) heating L-lactic acid in an atmosphere of inert gas such as nitrogen gas at atmospheric pressure or under reduced pressure;
(b) dissolving the resulting reaction solution in a heated state in methanol or ethanol and then filtering the solution;
(c) drying the filtrate under reduced pressure and dissolving it in acetonitrile, or directly adding the filtrate to acetonitrile;
(d) subjecting the resulting solution to chromatography on a reverse phase ODS or DS column previously equilibrated with 25 % aqueous acetonitrile at pH 2 to 3 and then eluting it with 30 to 50 % aqueous acetonitrile at pH 2 to 3;
(e) after elution, obtaining the malignant tumor cell growth inhibitor for mammals including humans, which is a fraction eluted with 70 % or more aqueous acetonitrile at pH 2 to 3.

This inhibitor was against human uterine cervical region strain cells, humans nasal pharynx cancer strain cells, hydro cavity bottom cancer strain cells, mouse lung cancer cells, rabbit hepatoma-derived strain cells, Yoshida sarcoma, human stomach cancer, thyroid cancer, lung cancer and uterine cancer.

The present inventors found that given a major working mechanism of inhibition of the activity in the glycolytic pathway significantly promoted in malignant tumor cells as compared with normal cells, poly-L lactate with a degree of condensaiton of 3 to 23, preferably 9 to 19 exhibits a strong anti-tumor cell growth inhibitory action on colon cancer, esophagus cancer and/or breast cancer and further has a significant constitution-improving action including alleviating pain, in particular cancerous sharp pains accompanying malignant tumors.

The object of the present invention is to provide a L-lactate oligomer having a degree of condensaiton of 3 to 23, preferably 9 to 19 found to have this new distinctive effect as an anti-malignant tumor agent for mammals including humans.

This involves selection of oral and parental administration depending on situations such as treatment of malignant tumors and prevention of incidence as well as combined therapy with existent anti-tumor therapy for attaining the maximum effect, thus contributing to expansion of the conventional therapeutic methods against malignant tumors and improvement of therapeutic effects.

To achieve the object described above, the anti-malignant tumor agent of the present invention comprises a mixture of cyclic and linear poly L-lactate oligomers having degrees of condensation of 3 to 23, which is prepared by dehydration condensation of L-lactic acid in a nitrogen atmosphere under stepwise reduced pressure and at an increasing temperature, drying ethanol- and methanol-soluble components in the resulting reaction solution under reduced pressure, subjecting the product to chromatography on a reverse phase ODS column, and after dissolution in 25 to 50 % aqueous acetonitrile at pH 2.0, dissolving said mixture of poly L-lactates as a fraction dissolved in 100 % acetonitrile at pH 2.0. This anti-malignant tumor agent can be utilized as an anti-tumor agent against malignant neoplasms including cancers.

According to the present invention, there is provided an anti-malignant tumor agent used in malignant neoplasms including cancers in mammals including humans, which is highly biocompatible and recognized to have an anti-tumor effect even in oral administration.

For a better understanding of the invention, and to show how the same may be carried into effect, reference will now be made, by way of example, to the accompanying drawings, in which:-
Fig. 1 shows the result of mass spectrometric analysis of L-lactate oligomers.
Fig. 2 shows a model for the molecular structure of an L-lactate oligomer at a low degree of polymerization.
Fig. 3 shows a model for the molecular structure of an L-lactate oligomer at a high degree of polymerization.
Fig. 4 shows expression of 7A6 antigen in TF-1 cells in 24-hours culture after CPL was added.
Fig. 5 shows the effect of CPL on the activity of pyruvate kinase (PK).
Fig. 6 shows the effect of CPL on the activity of lactate dehydrogenase (LDH).

To serve in actual use, L-lactate oligomers with degrees of condensation of 3 to 23 were separated, purified, neutralized with an alkali and dried under reduced pressure to give raw powder, which was then aseptically dissolved or suspended at a predetermined concentration in a suitable solvent to prepare an injection.

When used as an oral agent, the raw powder treated in the same manner as described above can also be used as such, but in consideration of the properties of this substance, a stabilizer such as calcium lactate, calcium carbonate, mannitol or sorbitol is usually added.

Further, it can be administered in the form of a mixture with other pharmaceutically acting substance. The product can be formed into pharmaceutical forms such as powder, granules, tablets, sugar-coated tablets, capsules, suspensions and emulsions.

Hereinafter, the present invention is described in more detail by reference to the Examples, which however are not intended to limit the present invention.

### [Production Example]

500 ml L-lactic acid is introduced into a separable flask placed on a mantle heater and stirred with a nitrogen gas passing at a rate of 500 ml/min.

While effluent water is introduced into a flask equipped with a reflex condenser, free water is distilled away by heating for 3 hours until the temperature reaches 145 °C. Then, the atmosphere is reduced to 20 kPa, and the reaction solution is heated for 3 hours.

Thereafter, the reaction solution is heated at 0.7 kPa for 3 hours until the temperature reaches 170 °C.

Finally, the solution is heated for 1.5 hours until the temperature reaches 190 °C, to give the reaction product, L-lactate oligomers.

The resulting L-lactate oligomers are kept at 100 °C, and 100 ml ethanol and then 400 ml methanol are added dropwise thereto and cooled by leaving the solution.

The L-lactate oligomers are added to 500 ml methanol, then vigorously stirred, and allowed to stand.

The L-lactate oligomers are purified by filtration, and the filtrate is dried under reduced pressure and dissolved in acetonitrile to adjust the total volume to 200 ml (stock solution).

This stock solution is applied to a previously equilibrated reverse phase ODS column (TSK gel ODS-80^{TM}) and eluted stepwise with 30 %, 50 % and 100 % acetonitrile (pH 2.0) containing 0.01 mol hydrochloric acid, to give lactate oligomers (degree of condensation: 3 to 23) as a fraction eluted with 100 % acetonitrile.

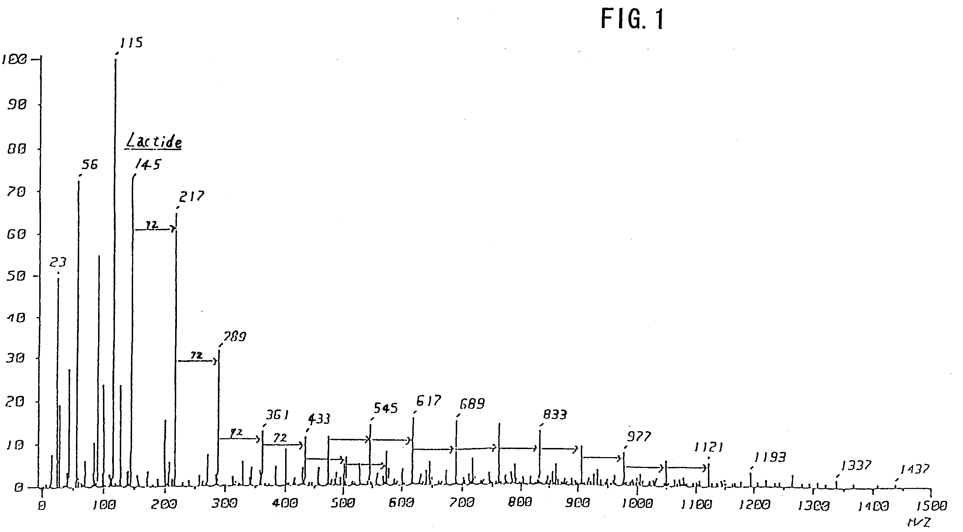

The result of mass spectrometric analysis of the L-lactate oligomers is shown in Fig. 1. As shown in Fig. 1, the synthesized L-lactate oligomers contain cyclic and linear oligomers in an about 7 : 3 ratio, which are polycondensates having degrees of polymerization (Z) of up to 23. The ratio of the cyclic to linear oligomers in the L-lactate oligomers is shown in Table 1.

**[Table 1]**

| Ratio of cyclic to linear L-lactate oligomers | |
|---|---|
| Degree of polymerization Z | Ratio (%) cyclic type : linear type |
| Z=2 | 63 : 37 |
| Z=3 | 70 : 30 |
| Z=4 | 81 : 19 |
| Z=5 | 64 : 36 |
| Z=6 | 71 : 29 |
| Z=7 | 86 : 14 |
| Z=8 | 83 : 17 |

The chemical structural formulae of the cyclic and linear condensates are as follows:
Structure of the cyclic condensate (C₃H₄O₂)X 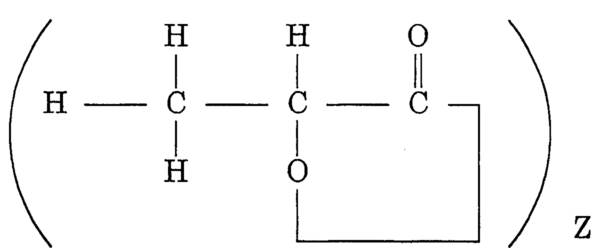 Structure of the linear condensate (C₃H₄O₂)X-H₂O

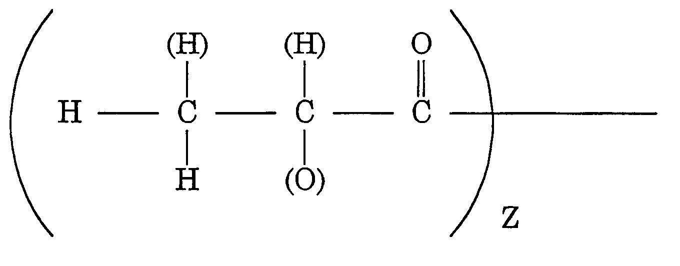

There is a certain reversible equilibrium relationship between the cyclic and linear oligomers, and in light of e.g. a diversity of biological activities of L-acetate oligomers and the fact that the anti-tumor effect is brought about by a combination of fractions having different degrees of condensation, there is less significance in separating the cyclic and linear condensates from each other before use.

Accordingly, the present inventors named the L-lactate oligomer "cyclic poly-lactate (CPL)".

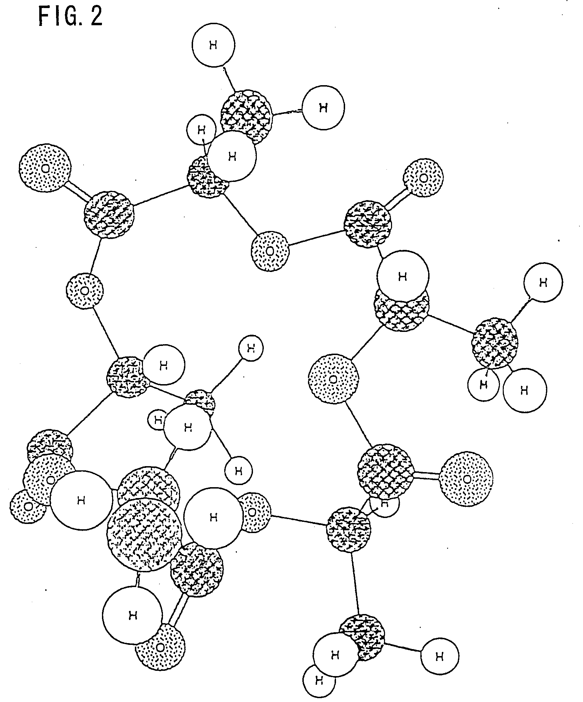

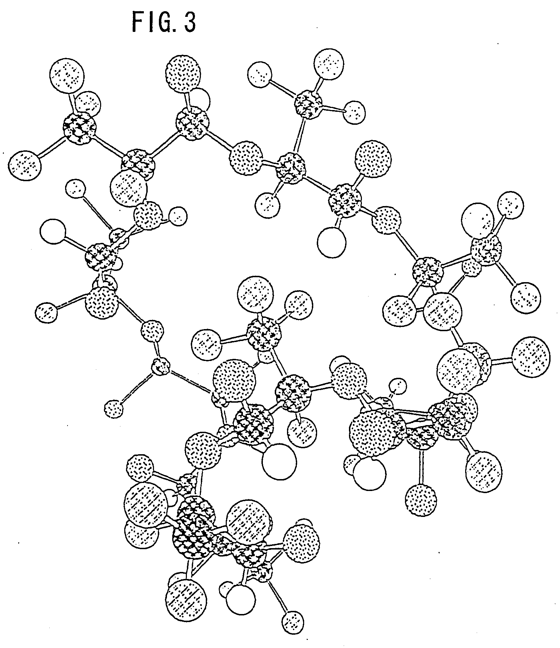

On the basis of energy calculation by molecular dynamics using a computer model, the CPL molecule has a hollow zigzag closed-ring cyclic structure at a low degree of condensation (Fig. 2), but at a high degree of condensation, the closed-ring long elliptic ring shows a C-shaped, bent zigzag structure (Fig. 3), and the molecule assumes both cyclic and linear characters. The linear oligomer molecule shows a structure linked in a linear form to extend or fix it in a 2-dimensional direction.

### Preparation Example 1

The L-lactate oligomer (CPL) was neutralized with 1 mol/m³ sodium hydroxide, dried under reduced pressure, dissolved at a concentration of 100 mg/ml in propylene glycol of 70 to 80°c, sterilized by filtration with 0.45 µm filter, and introduced into vials by aseptic pipetting (15 ml per vial) to prepare an injection.

### Preparation Example 2

The L-lactate oligomer (CPL) was neutralized with 1 mol/m³ sodium hydroxide and dried under reduced pressure in the same manner as described above to give 1800 g sample, and after 500 g sorbitol and 200 g calcium carbonate were added, the sample was solidified and ground to give impalpable powder which was then aseptically introduced into vials to prepare oral powder.

### Toxicity test 1

For confirmation of safety, CPL was administered intravenously into male ICR strain mice for 14 consecutive days at doses of 15, 30 and 60 mg/kg, respectively. There was no dead mouse in any administration group during the administration period and during a later observation period, and no change was observed in behaviors including motility cooperation, evacuation, urination and physical conditions, and the body weight changed favorably, indicating the increase in the body weight during the administration period was in the range of 14.4 to 14.9 g for the low, middle and high administration groups, as compared with 14.6 g for the solvent control group.

### Toxicity test 2

Supposing clinical adaptation to humans, two dogs were administered with 49 mg/kg CPL for 15 consecutive days by intravenous drip infusion (60 drops/min.), to evaluate its safety. During the administration period and during a later observation period, there were neither abnormal symptoms nor death, and physical conditions such as body temperature and heartbeats were normal.

The results in a hematological test were also within the normal range, and findings suggesting anemia, inflammations, and disturbances in hepatic functions and renal functions were not recognized, and no abnormality in each organ was recognized visually and histologically. The measurement results in major items before and after administration of CPL are shown in Table 2.

**[Table 2]**

| Measurement results in major items before and after administration of CPL | | |
|---|---|---|
| Major measurement items | Before administration | After intravenous drip infusion for 15 days |
| Body weight (kg) | 13.0-16.0 | 15.0-17.5 |
| Number of erythrocytes (per 1 mm³) | 4,970,000-5,230,000 | 4,530,000-4,830,000 |
| Hemoglobin level (g/dl) | 12.8-13.0 | 13.1-13.9 |
| Number of leukocytes (per 1 mm³) | 9,100-12,800 | 10,600-11,300 |
| Number of platelets (per 1 mm³) | 143,000-156,000 | 105,000-113,000 |
| GPT activity (U/1) | 15-23 | 17-26 |
| GOT activity (U/1) | 29-45 | 24-43 |
| BUN level (mg/dl) | 14-32 | 14-17 |

### Toxicity test 3

For confirmation of the safety of CPL upon oral administration, male and female ICR strain mice were once administered orally with CPL at a dose of 2000 mg/kg and observed for 2 weeks. After administration, there occurred neither abnormal symptoms nor death, and the body weight changed in a similar way to the solvent control group. No abnormality was recognized pathologically, visually and histologically.

### [Influence 1 on malignant tumor cell growth in vitrol

CPL was added to human stomach cancer cells AZ521 and colon cancer cells DLD1, and these cells were cultured for 24 hours and 72 hours, respectively, and the influence of CPL on growth of both the cells was determined by the MTT method. As a result, significant growth inhibition was observed after 72 hours of culture, as follows: As compared with the control, the degrees of growth of AZ251 and DLD1 given 1.9 mg/ml CPL were 48 % and 47 % respectively, and the degrees of growth ofAZ251 and DLD1 given 7.5 mg/ml CPL were 7 % and 12 % respectively.

### [Influence 2 on malignant tumor cell growth in vitrol

CPL was added to humans leukemia cells HL60 and TF-1, and the number of living cells was measured daily with trypan blue for 10 days after culture was initiated, to evaluate the influence of CPL on growth of both the cells. By addition of 0.2 mg/ml CPL, the significant inhibition of growth of both the cells was observed from the first day of culture.

Further, it was confirmed by electrophoresis that their extracted DNA had been fragmented, and in examination with monoclonal antibody Apo2.7 (2.7A6A3 clone), generation of 7A6 antigen was recognized, thus suggesting that an apoptosis induction-mediated mechanism is involved in the inhibition of growth of tumor cancer cells by CPL.

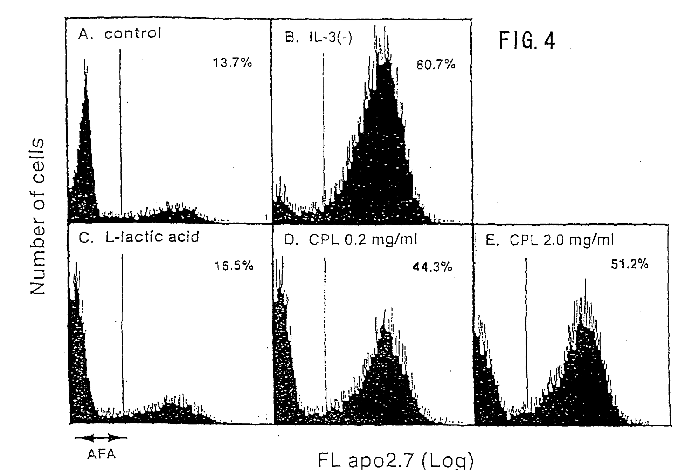

Fig. 4 shows generation, as determined by flow cytometry, of 7A6 antigen in TF-1 cells cultured after CPL was added.

### [Influence of malignant tumor cells on energy metabolism]

Vigorously proliferating tumor cells depend highly on the anaerobic glycolytic pathway in order to maintain the supply of much energy. Accordingly, the influence of CPL on key enzymes in the anaerobic glycolytic pathway, that is, pyruvate kinase (PK) and lactate dehydrogenase (LDH), was measured.

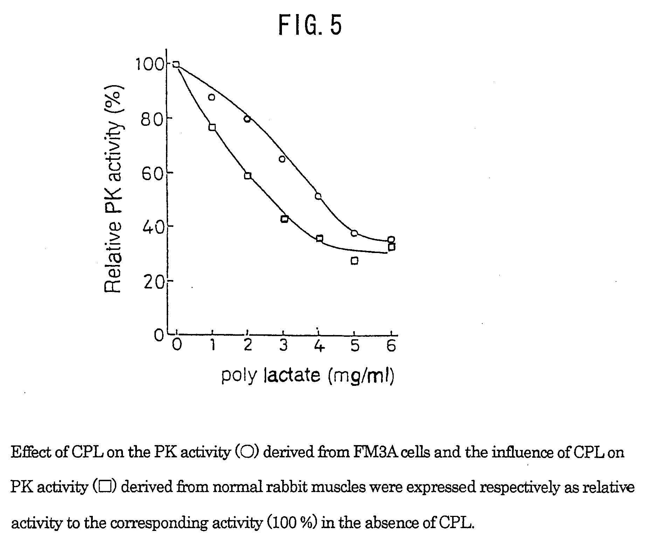

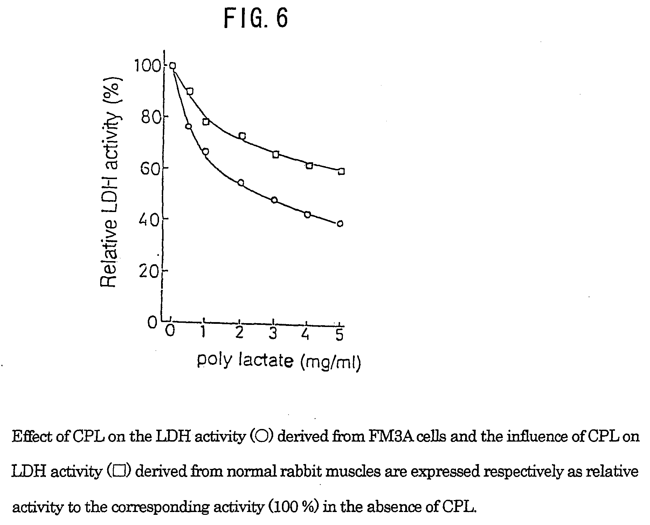

As shown in Figs. 5 and 6, CPL significantly inhibits the activities of PK and LDH from FM3A ascitic cells derived from mouse breast cancer, and 50 % inhibitory concentration (IC₅₀ was 4 mg/ml for PK and 2.5 mg/ml for LDH.

In measurement in the anaerobic glycolytic pathway, it was found that by addition of 8 mg/ml CPL, the amount of lactate formed was lowered to 50 % of the control.

### [Influence 1 on malignant tumor cell growth in vivo]

Mice having FM3A cells implanted intraperitoneally therein (2×10⁶ cells/mouse) were administered intraperitoneally with CPL at a dose of 4 mg/mouse from the day after implantation, and the number of cancer cells in their ascites was measured with time. It was recognized that on the 12th day after implantation, the number of cancer cells had been increased to {78.1±13.4}×10⁷ cells in the control group, while in the group given CPL, the number of cells was decreased to {20.2±3.8}×10⁷, that is, the cancer cells were decreased by 25 %. Cytomorphological changes such as disappearance of cytoplasmic protuberance and reduction in nuclear chromatin were observed.

### [Influence 2 on malignant tumor cell growth in vivo]

New Zealand white male rabbits having cancroid cells VX2 implanted in the liver through the peritoneal cavity opened under anesthesia with pentobarbital were administered via veins or directly via hepatic arteries with CPL or an anticancer drug adriamycin (ADM), and the sizes of their tumors were measured and examined by comparison. The inhibitory effect of CPL on proliferation of VX2 cancer is shown in Table 3.

**[Table 3]**

| Inhibitory effect of CPL on proliferation of VX2 cancer | | | | | | |
|---|---|---|---|---|---|---|
| | | | VX2 cancer | | | |
| Administation route route | Group | Animal No. | Tumor size 1) | | Rate of proliferation 2) | Induced mortality 3) |
| | | | Before admnistration | After admnistration | | |
| Intravenous (IV) | Control 5% sugar solution | 1 | 225 | 550 | 244 | <10% |
| | | 2 | 255 | 572 | 224 | <10% |
| | | 3 | 360 | 440 | 1.22 | <10% |
| | CPL 15mg/Kg | 4 | 270 | 432 | 1.6 | <10% |
| | | 5 | 360 | 345 | 0.96(reduction) | <10% |
| | | 6 | 180 | 600 | 3.33 | <10% |
| | Adrianycin 0.5mg/Kg | 7 | 180 | 286 | 1.59 | <10% |
| | | 8 | 300 | 280 | 0.93 (reduction) | <10% |
| | | 9 | 342 | 391 | 1.14 | <10% |
| Into hepatic arteries(TAI) | CPL 6mg/per animal | 10 | 150 | 120 | 0.8 (reduction) | complete necrosis |
| | | 11 | 270 | 104 | 0.39 (reduction) | complete necrosis |
| | | 12 | 340 | 264 | 0.78 (reduction) | complete necrosis |
| | Adrianycin 6mg/per animal | 13 | 180 | 180 | 1 | >90% |
| | | 14 | 270 | - | -(dead) | - |
| | | 15 | 270 | 330 | 1.22 | complete necrosis |

| | | | | | | |
|---|---|---|---|---|---|---|
| 1) Length × width (mm²) | | | | | | |
| 2) (Size of cancer after administration)/(size of cancer before administration) | | | | | | |
| 3) Pathohistological judgment | | | | | | |

As shown in Table 3, the inhibitory effect on tumor growth, which is significant as compared with the control group and similar to the group given ADM, was observed in both the group given CPL via veins or into hepatic arteries. Necrotic change of the tumor is also recognized pathohistologically.

### Effect of CPL on survival period of mice carrying cancer

Mice having FM3A cells implanted intraperitoneally therein (2×10⁶ cells/mouse) were administered intraperitoneally with CPL in a dose of 4 mg/mouse from the day after implantation, and the effect of CPL on the survival period of the mice carrying the cancer was observed.

The result indicated that the survival period of 50 % of the mice in the untreated group was 16 days and all the mice died in 17 days after the cancer cells were implanted. The control group given the solvent showed no significant difference in the survival period from that of the untreated group. The survival period of 50 % of the mice in the group given CPL was 31 days, thus indicating a significant prolongation of the survival period.

### [Effect of CPL on prevention of transformation]

5-week-old C57BL strain mice, made deficient in cancer suppressing gene p53 by genetic recombination, were orally administered with 50 mg/kg CPL at the interval of 3 times/week for 20 weeks, and transformation rate and mortality rate were evaluated. It is reported that a wide variety of malignant tumors such as malignant lymphoma, vascular sarcoma, bone sarcoma and testicle tumor are generated in these mice in age from about 10 weeks, and about 70 % of these mice generate malignant tumors in age from 6 months.

The mice in the control group died of malignant tumors after 8 weeks, and after 20 weeks, only 1 mouse (10 %) survived, while in the group given CPL, 5 out of 10 mice (50 %) survived. The onset of malignant tumors was pathologically observed in every dead mouse, and no significant difference was observed between the control group and the group given CPL in respect of the type and distribution of generated malignant tumors.

### [Effect of CPL on the immune activity of mice carrying cancer]

C57BL strain mice having malignant melanoma cells B16 implanted intravenously therein were administered with CPL orally (500 mg/kg) or intravenously (10 mg/kg) for 7 days, and upon initiation and termination of administration, NK activity (activity of natural killer cells in impairing the tumor cells) was measured. The effect of CPL on the NK activity in the mice carrying B16 malignant melanoma is shown in Table 4.

**[Table 4]**

| [Effect of CPL on NK activity in mice carrying B16 malignant melanoma] | | |
|---|---|---|
| Group | Day 0 after administration | Day 7 after administration |
| Control | 15.0 ± 3.7 | 4.9 ± 3.9 |
| 500mg/Kg, p.o. | 12.5 ± 2.9 | 7.4 ± 10.6 |
| 10mg/kg, i.v. | 13.2 ± 1.9 | 15.7 ± 3.2** |
| Effector cell/Target cell = 100/i | | |
| The values show NK activity (%)±S.C | | |
| There is a significant difference from the control group: **P<0.01 | | |

As shown in Table 4, the NK activity in the control group on Day 7 was reduced to about 30 %, while no reduction in the NK activity was recognized in the group given CPL. In simultaneously conducted pathologically examination, it was found that the number of tumor colonies appearing in the lung in the treated group was lower than that of the control group.

### [Analgesic effect of CPL]

5-week-old ddy strain mice were used to evaluate the effect of CPL on pain (agony) reaction induced by acetic acid. CPL was administered (500 mg/kg) orally twice in 3 hours and 1 hour before administration of acetic acid, or injected subcutaneously once (50 mg/kg) before administration of acetic acid, and upon intraperitoneal injection of 0.7 % acetic acid, the number of pain (agony) reactions was determined. By preliminary administration of CPL, a reduction in the number of pain (agony) reactions was recognized. Particularly in the group given CPL by subcutaneous injection, the number of pain reactions was reduced to 52 % of that of the control group.

### [Clinical therapeutic effect by intravenous drip infusion]

Upon consent and request of patients and their families, about 50 patients with severe cancers diagnosed as non-operable terminal cancers, remaining cancers or metastasized cancers after operation, or recurring cancers, received cancer treatment using drip infusion of CPL prepared in Preparation Example 1.

Standard therapy consisted of drip intravenous injection of 200 mg CPL/human/day for 20 days as 1 sequence. As the drip, 20 ml of the injection was mixed with, and dissolved in, 500 ml solution for blood transfusion such as glucose solution or physiological saline and administered for 3 hours.

It was found that among the various primary cancers treated with the CPL injection, the colon cancer, esophagus cancer and breast cancer underwent the most significant growth inhibitory effect, and further the growth inhibitory effect was observed on recurrence of these cancers or in their metastasized foci.

In particular, significant anti-cancer effect was recognized on the remaining cancers in patients (about 14 person) just after surgical excision or radiotherapy, and amelioration was confirmed in about 80 % of these patients, and it was clinically judged that among these patients, 8 persons were almost healed.

According to administration of this injection, not only the anti-tumor effect but also improvement of nutritional conditions,
improvement of subjective symptoms such as feeling of exhaustion, and early recovery from sides effects of radiotherapy and chemotherapy, such as a reduction in leukocytes, anemia and disturbances in hepatic functions are recognized, and these results are considered to suggest that use of CPL in combination with existent anti-tumor therapy can be expected to achieve higher anti-cancer effects.

In the standard administration method, there were cases where transient fever appeared as side effect upon first intravenous drip infusion, but the fever was light and rapidly relieved by simultaneous use of succinate dehydrocortisone in a dose of 100 to 200 mg for each administration.

Further, in the case of drip infusion of 2000 mgxtwice/day, which is twice as high as the standard dose, or in the case of administration for consecutive days in 3 consecutive months, no side effects were recognized except for those described above.

### Case 1: Colon cancer

The intravenous drip infusion of the present injection by the standard therapy was conducted for 2 years on a 60-year-old male having transverse-colon cancer accompanied by invasion into the spleen and adhesion, who after surgical excision and bypass operation, was observed to have the remaining cancer. As a result, the inhibition of proliferation of the cancer was recognized in examination with X rays and supersonic echo, and further the inhibitory effect on metastasis and recurrence was also recognized. In about 3 to 10 days after the therapy was initiated, subjective findings such as disease, feeling of exhaustion, easily caused fatigue and nausea, recovery of appetite, an increase in the body weight, and improvement of nutritional conditions were recognized, and he was freed from psychological anxiety and spiritual desolation, and the improvement of living activity was recognized.

### Case 2: Lung cancer metastasized from colon cancer

The intravenous drip infusion of the present injection by the standard therapy was conducted on a 60-year-old male who after surgical excision of colon cancer, had multiple cancer metastasized in the lung diagnosed as anaerobic lung which was found to have been developed for about 3 to 4 months by a prognosis.

By image diagnosis, it was recognized that cancer foci remained in the lung, but lung functions were very good during this treatment for about 3 years, and neither magnification of the cancer foci nor metastasis in other organs was recognized, and the significant effect of prolonging life was observed. In this case, appetite and nutritional conditions were also improved, subjective symptoms such as sharp pain were not observed, and the living activity was in good conditions.

### Case 3: Esophagus cancer

The intravenous drip infusion by the standard therapy was conducted on a 50-year-old male who underwent surgical excision after diagnosis of esophagus cancer, recognized to have hematemesis along with poor appetite and difficult swallowing in 17 months after the operation, and found in endoscopy to have recurring esophagus cancer and stricture in the sutured portion.

On the 10th day after the therapy was initiated, he could take food, and a reduction in the cancer and re-opening of the stricture were recognized in endoscopy. In observation of the progress thereafter, good conditions are maintained.

### Case 4: Breast cancer

The intravenous drip infusion by the standard therapy was conducted on a 42-year-old female who in the 3rd year after operation of breast cancer, had sharp pain in hip-bone and rib diagnosed as metastasized cancer in bone scintigraphy. Because this patient had undergone chemotherapy for 4 months after diagnosis of the metastasized cancer, a reduction in the number of leukocytes (2900/µl), anemia (3,840,000 µl erythrocytes_{/}µl and 10.7 g hemoglobin/dl) and hepatic function disturbance (ALT level: 102 IU/l) were recognized when the administration of the present injection was initiated.

After the fourth administration, the number of leukocytes was increased to 8100/µl and the hepatic function was improved to an ALT level of 21 IU/1, and after the seventh administration, subjective symptoms such as feeling of exhaustion, breast-pang and bone pain disappeared. In 2 years after 1 sequence of drip infusion was finished, there are no subjective symptoms without progress of the cancer.

The present injection exerts a relatively strong effect on the cancers in gynecology thereby bringing about long survival and significant effect in many cases. Upon intravenous drip infusion, many patients with breast cancer complain of a sense of incongruity, thus suggesting its direct action on cancer lesions, and topical administration to the cancer such as selective intraarterial administration is considered to be a useful method applicable to the present agent.

By administration of the present agent, the alleviation of complicated diabetes and hyperlipemia was confirmed as its effect on the abnormal metabolism of sugars and lipids. These actions, along with the above-described effects of improving hepatic functions etc., are considered to act advantageously on treatment of malignant tumors. Further, the combination of the intravenous drip infusion with the oral powder prepared in Preparation Example 2 was confirmed to be effective in enhancing and keeping the effect of this injection.

### [Clinical therapeutic effect by oral administration]

The oral administration of CPL was conducted basically by administering the powder prepared in Preparation Example 2 in three portions at a dose of 6 to 10 g/day, and the prescriptions for the dosage and administration method were altered depending on symptoms. The same anti-tumor effect as by the intravenous administration described above was recognized, and in particular the oral administration can be controlled without requiring hospitalization, thus contributing to alleviation of mental and economical burden on the patient.

As feeling of fullness was recognized as side effect at an initial stage of administration, but thereafter the symptoms disappeared without any particular treatment. Other subjective and objective abnormalities have never been recognized.

### Case 1

A 66-year-old male who after removal of the left upper lobe because of the lung cancer (adenocarcinoma), was recognized 2 years later to have recurrence of cancerous pleuritis and pericarditis, so he received general administration of 5FU to the whole body and thoracic cavity, and upon drainage by pricking the pericardium, he also received administration of cisplatin into the thoracic cavity and pericardium, but while accompanying difficult respiration, liquid storage was not alleviated thus making drainage necessary for almost consecutive days.

From 2 weeks after the above treatment was initiated, the oral administration of CPL in a dose of 6 g/day was initiated. The liquid storage was gradually decreased, and about 6 months later, the liquid storage in the thoracic cavity and pericardium became absolutely unrecognizable, and the progress thereafter was good. Symptoms such as poor appetite, general exhaustion, nausea and emesis recognized upon recurrence came to be alleviated after administration of CPL, and after about 2 weeks of administration, these symptoms were almost alleviated.

### Case 2

A 69-year-old male with jaundice, poor appetite and a sense of incongruity in an upper berry was diagnosed as suffering from pancreas cancer and underwent chemotherapy with UFT and radiotherapy. Along with the chemotherapy, oral administration of CPL was initiated. After about 1 week of administration, his appetite was recovered, and after 2 weeks, subjective symptoms including jaundice were alleviated.

As shown in the cases described above, the same anti-tumor effect as in intravenous administration is also recognized in oral administration of CPL, and further there is also the effect of improving subjective findings such as increase in appetite.

The present invention shows that the poly L-lactate (CPL) having degrees of condensation of 9 to 19 exhibits a superior inhibitory action on proliferation of malignant tumors and simultaneously suppresses recurrence and metastasis of these tumors, thus serving not only as an injection and an oral drug but also for effective therapy against these malignant tumors. These effects were confirmed both in vitro and in vivo.

Because CPL is a low condensate of L-lactate derived from biological components, it has the distinctive features that its biocompatibility is extremely high, side effects are hardly observed even in the most severe intravascular administration, and the anti-tumor effect is recognized even in oral administration.

That is, the administration method can be selected depending on the conditions of the case including the period of the disease, and it can be expected that the effect is maintained or enhanced by the combination described above.

Upon combination with existent chemotherapy with anti-tumor agent, CPL can be expected to demonstrate the maximum therapeutic effect while reducing the side effects of the existing chemotherapy, such as a reduction in leukocytes and hepatic function disturbances. In addition, CPL can be successively given safely and easily for a prolonged period of time and may be subjected to prevention of transformation as experimentally suggested.

Concerning the anti-tumor effect of CPL, vigorously proliferating tumor cells, unlike normal cells, depend highly on the anaerobic glycolytic pathway in order to maintain their extremely high metabolic activity and their requirement for much energy. And it was revealed that CPL has a significant inhibitory effect on proliferation of tumor cells by inhibiting such anaerobic glycolytic pathway in the tumor cells.

In particular, the strong inhibition of the activities of pyruvate kinase and lactate dehydrogenase, which are key enzymes in the anaerobic glycolytic pathway, was recognized, thus suggesting that this action is one function of CPL.

As the result of this inhibitory action, CPL is observed to induce the apoptosis of tumor cells. It is recognized that additional functions of CPL lie in the immune activating action by activation of NK cells as well as in the effect of improving hepatic and digestive functions via metabolism of glycolipids. By virtue of these diverse actions, CPL is estimated to be one factor showing a relatively broad anticancer spectrum without causing severe side effects, unlike existent anti-malignant tumor agents with a strong action of impairing cells.

In clinical experience, it was revealed that CPL has not only the anti-tumor effect but also excellent effects of alleviating malnutrition including want of appetite and reduction of the body weight, physical symptoms such as anemia, sharp pain, feeling of exhaustion etc., and reduced living activity necessitating mental and physical aids.

In particular, the usefulness of CPL in alleviating nutritional disturbances in patients with cancers, such as reduction in immune ability and cachexia, can be mentioned as an important effect, and this effect can be expected not only in patients with malignant tumors but also in patients with other diseases having similar symptoms.

As exemplified above, it is demonstrated that CPL has not only the anti-tumor effect but also the action of alleviating the symptoms of malignant tumors, thus serving as an effective means for improving the quality of life (QOL) of patients, and taking everything into consideration, it can be said that CPL is a medicine having new possibility as compared with conventional anti-malignant tumor agents.

## Claims

1. A method of preparing an anti-malignant tumor agent comprising a mixture of poly L-lactates that are cyclic and linear oligomers having degrees of condensation of 3 to 23 preferably 9 to 19, the method comprising dehydration condensation of L-lactic acid in a nitrogen atmosphere under stepwise reduced pressure and at an increasing temperature, drying ethanol- and methanol-soluble components in the resulting reaction solution under reduced pressured, subjecting the product to chromatography on a reverse phase ODS column, and after elution with 25 to 50% aqueous acetonitrile at pH 2.0, giving said mixture of poly L-lactates as a fraction eluted with 100% acetonitrile at pH 2.0.

2. An anti-malignant tumor agent obtainable by the method of Claim 1.

3. An anti-malignant tumor agent comprising a mixture of poly L-lactates that are cyclic and linear oligomers having degrees of condensation of 3 to 21, preferably 9 to 19, and a pharmaceutically acceptable carrier.

4. An agent according to Claim 2 or 3 for use in treating disease.

5. Use of an agent according to Claim 2 or 3 for the manufacturing of a medicament for treating malignant neoplasms including cancers.

6. Use according to Claim 5 wherein the cancers are selected from colon cancer, esophagus cancer and/or breast cancer.

7. Use of an agent according to Claim 2 or 3 for the manufacture of a medicament for purposes selected from the group comprising alleviating pain, increasing appetite, alleviating exhaustion, improving hepatic functions, alleviating jaundice and/or strengthening the immune system.

8. Use of an agent according to Claim 2 or 3 for the manufacture of a medicament for alleviating nausea.

9. Use of an agent according to Claim 2 or 3 for the manufacture of a medicament for alleviating diabetes.

10. Use of an agent according to Claim 2 or 3 for the manufacture of a medicament for alleviating hyperlipemia.
